**Europäisches Patentamt**

**European Patent Office**

**Office européen des brevets**

(19)

(11) Veröffentlichungsnummer : **0 537 762 B1**

## (12) EUROPÄISCHE PATENTSCHRIFT

(45) Veröffentlichungstag der Patentschrift :
**13.12.95 Patentblatt 95/50**

(51) Int. Cl.[6] : **G01T 1/29**

(21) Anmeldenummer : **92117717.6**

(22) Anmeldetag : **16.10.92**

(54) **Verfahren und Vorrichtung zur Ermittlung der Dichteverteilung von ins menschliche Gewebe gebrachten Positronen**

(30) Priorität : **18.10.91 DE 4134435**

(43) Veröffentlichungstag der Anmeldung :
**21.04.93 Patentblatt 93/16**

(45) Bekanntmachung des Hinweises auf die
Patenterteilung :
**13.12.95 Patentblatt 95/50**

(84) Benannte Vertragsstaaten :
**DE FR GB NL**

(56) Entgegenhaltungen :
**EP-A- 0 172 929**
**EP-A- 0 449 690**
**US-A- 4 057 727**

(56) Entgegenhaltungen :
**IEEE TRANSACTIONS ON NUCLEAR SCIENCE, Bd. 33, Nr. 1, Februar 1986, New York, US, Seiten 464-467 ; H. Uchida et al. : "Design of a mosaic detector system for positron CT"**
**IEEE TRANSACTIONS ON NUCLEAR SCIENCE, Bd. 36, Nr. 1, Februar 1989, New York, US, Seiten 1006-1010 ; H. Iida et al. : "Design and evaluation of headtome-IV, a whole body positron emission tomograph"**

(73) Patentinhaber : **FORSCHUNGSZENTRUM JÜLICH GMBH**
**Wilhelm-Johnen-Strasse**
**D-52425 Jülich (DE)**

(72) Erfinder : **Schelten, Jakob, Prof.**
**Peter-Stommen-Strasse 10**
**W-5170 Jülich (DE)**
Erfinder : **Reinartz, Richard**
**Dechant-Kallen-Strasse 38**
**W-5163 Langerwehe (DE)**

EP 0 537 762 B1

**Beschreibung**

Die Erfindung bezieht sich auf ein Verfahren und eine Vorrichtung zur Ermittlung der Dichteverteilung von ins menschliche Gewebe gebrachten und dort in je zwei entgegengesetzt fliegende $\gamma$-Quanten zerfallenden Positronen, bei dem zwei gegenübergestellte, den Menschen einschließende $\gamma$-Detektoren derartige $\gamma$-Quanten signalisieren, ein an die $\gamma$-Detektoren angeschlossener Computer ein gleichzeitiges derartiges Signalisieren eines jeden $\gamma$-Detektors und daher zwei $\gamma$-Quanten, die aus demselben Positronenzerfall stammen, registriert, dieser Computer im Fall der Registrierung solcher aus demselben Positronenzerfall stammenden zwei $\gamma$-Quanten die Flugbahn dieser zwei $\gamma$-Quanten berechnet und damit eine Ortsinformation über das zugehörige zerfallene Positron ermittelt, dieser Computer solche Ortsinformationen speichert, die Positionen der $\gamma$-Detektoren derart verändert werden, daß aus der dann resultierenden Vielzahl solcher Ortsinformationen eine Verteilungsfunktion entsteht und der Computer aus dieser Verteilungsfunktion die Dichteverteilung der zerfallenen Positronen ermittelt. Ein derartiges Verfahren bzw. eine derartige Vorrichtung sind aus US-A-4,057,727 bekannt.

Das Verfahren wird Positronen-Emissions-Tomographie (PET) genannt und u.a. bei wissenschaftlichen Untersuchungen eingesetzt. Einem Menschen wird durch Injektion oder Inhalation ein Radiopharmazeutikum mit einem Positronemittierenden Isotop verabreicht. Die emittierten Positronen haben im menschlichen Gewebe Reichweiten von einigen mm bis zu einiger cm je nach ihrer Energie von typisch 1 bis 5 MeV und zerstrahlen, wenn sie zur Ruhe gekommen sind, in der genannten Weise.

Von den in bekannten PET-Scannern verwendeten Einzeldetektoren, die zu sich gegenüberstehenden Detektorbänken zusammengefaßt sind, wird nur ein kleiner Teil der emittierten Strahlung erfaßt. Es ist daher erforderlich, sie um das Meßobjekt zu drehen und vertikal zu verschieben, um in aufeinanderfolgenden Meßreihen einen großen Raumwinkel abzudecken. Dies führt zu zunehmender Meßdauer des Verfahrens, woraus eine unerwünschte Strahlenbelastung des Menschen resultiert.

Neben Einzeldetektoren zur Messung von $\gamma$-Quanten existieren inzwischen ortsauflösende Photomultiplier mit vorgeschaltetem Szintillator. Diese besitzen eine Detektorfläche von 10 cm $\phi$ und erreichen eine Ortsauflösung von 1 mm, siehe zum Beispiel IEEE TRANSACTIONS ON NUCLEAR SCIENCE Bd. 33, Nr. 1, Februar 1986, NEW YORK US Seiten 464 - 467
UCHIDA ET AL. 'Design of a mosaic BGO detector system for positron CT.'

Typischerweise werden $10^8$ Zerfallsereignisse im Verlauf einer Messung ermittelt, um zur Dichteverteilung der Positronen-Zerfallsereignisse zu gelangen. Ein Einsatz von derartigen Photomultipliern anstelle von Einzeldetektoren würde die Erfassung von $10^8$ Zerfallsereignissen wesentlich beschleunigen und damit die Meßdauer um Größenordnungen reduzieren. Wird jedoch von etwa 16 Winkeleinstellungen, um die Photomultiplier um das Meßohjekt zu drehen, und von 4 vertikalen Verschiebungspositionen ausgegangen, so entsteht ein Speicherplatzhedarf von wenigstens 425 MByte, ohne daß nach Abspeicherung der Daten irgendetwas an Informationen über die Dichteverteilung gewonnen worden wäre.

Die in der Positronen-Emissions-Tomographie üblicherweise eingesetzten Computer sind jedoch weder in der Lage die beim Einsatz von Photomultipliern entstehende Datenflut on-line in der bisherigen Weise zu verarbeiten, noch eine solche Datenmenge abzuspeichern.

Es ist Aufgabe der Erfindung, die Meßdauer bei der Positronen-Emissions-Tomographie zu verkürzen, um eine Verringerung der Strahlenbelastung zu ermöglichen, ohne daß dabei die Investitionskosten für solche Tomographen erheblich vergrößert werden.

Erfindungsgemäß werden bei einem Verfahren bzw. bei einer Vorrichtung der eingangs genannten Art als $\gamma$-Detektoren zueinander parallel ausgerichtete ortsauflösende Photomultiplier eingesetzt und dem Speicher des Computers nur ein per Zufallsgenerator ausgewählter Teil an Ortsinformationen zugeführt, wobei dieser Teil an Ortsinformationen so groß gewählt ist, daß bei einer On-line-Datenspeicherung dieser Ortsinformationen die Leistungsfähigkeit des Computers nicht überschritten wird.

Um den Informationsverlust zu minimieren, wird pro durch den Computer berechnete Flugbahn per Zufallsgenerator eine Koordinate dieser Elugbahn, hier Ereigniskoordinate genannt, willkürlich gewählt, wobei die zugelassenen Zufallsgeneratorzahlen so eingeschränkt werden, daß die Ereigniskoordinaten im oder nahe beim menschlichen Gewebe liegen.

Ein einfaches und schnelles Speichern der Ereigniskoordinaten wird dadurch erreicht, daß der Ereigniskoordinatenraum in einzelne Raumelemente zerlegt wird, jedes Raumelement einer Speicherplatzadresse zugeordnet wird, eine Ereigniskoordinate in einem solchen Raumelement durch Inkrementierung um eins des zugeordneten Speicherplatzes gespeichert wird und so eine gespeicherte Ereigniskoordinatenverteilung $B_i$ ensteht. Um hieraus gemäß

$$\sum_k A_{ik} \cdot E_k = B_i$$

die Dichteverteilung $E_k$ der zerfallenen Positronen zu berechnen, wird das Auflösungsvermögen bei dieser Vorgehensweise näherungsweise analytisch oder duch Computersimulation berechnet oder experimentell ermittelt, indem die Ereigniskoordinatenverteilung eines einzelnen Positronenstrahlers, der zu diesem Zweck anstelle des Menschen zwischen die Photomultiplier gestellt wird, gemessen wird.

Die Auswertung der Ereigniskoordinatenverteilung erfolgt beispielsweise, indem die Dichteverteilung der zerfallenen Positronen $E_k$ iterativ gemäß

$$Y_{\underline{k}}^{(n+1)} = \alpha \cdot Y_{\underline{k}}^{(n)} \left( \frac{B_{\underline{k}}}{\sum_{\underline{j}} A_{\underline{k}\underline{j}} \cdot Y_{\underline{j}}^{(n)}} \right)^{\eta}$$

$0,1 < \eta < 10$

beginnend mit

$$Y_{\underline{k}}^{(1)} = \frac{|B_{\underline{k}}|}{|\sum_{\underline{j}} A_{k\underline{j}} \cdot B_{\underline{j}}|} \, B_{\underline{k}}$$

berechnet wird.

Eine Beschleunigung dieser Auswertung wird erreicht, indem die Summe

$$S_{\underline{k}} = \sum_{\underline{j}} A_{\underline{k}\underline{j}} \cdot Y_{\underline{j}}^{(n)}$$

näherungsweise in eine Summation über die diagonalen Elemente und in eine Mittelwertbildung über die verbleibenden Elemente zerlegt wird, d.h.

$$S_{\underline{k}} = \sum_{|\underline{j}-\underline{k}| \leq m} A_{\underline{k}\underline{j}} \cdot Y_{\underline{j}}^{(n)} + \sum_{|\underline{j}-\underline{k}| > m} \langle A_{\underline{k}} \rangle \cdot Y_{\underline{j}}^{(n)}$$

wobei

$$\langle A_{\underline{k}} \rangle = \frac{\sum_{|\underline{j}-\underline{k}| > m} A_{k\underline{j}}}{\sum_{|\underline{j}-\underline{k}| > m} 1}$$

Zur Durchführung des Verfahrens dient eine Vorrichtung, bei der als $\gamma$-Dektoren zueinander parallel aus-

gerichtete, ortsauflösende Photomultiplier eingesetzt sind und ein Zufallsgenerator derart mit dem Rechenwerk verbunden ist, daß durch den Zufallsgenerator ein Teil der Ortsinformationen auswählbar ist, so daß lediglich ein Teil der Ortsinformationen im Speicher on-line speicherbar ist.

Das erfindungsgemäße Verfahren wurde durch ein Simulationsexperiment überprüft.

Fig. 1 skizziert die Meßsituation mit zwei ortsauflösenden Photomultipliern $D_a$ und $D_b$, die ein würfelförmiges Meßobjekt einschließen.

Fig. 2 zeigt Meßwerte $B_i$ resultierend aus Zerfällen $E_k$ und das hieraus berechnete Ergebnis $Y_k$.

Fig. 3 veranschaulicht eine zur Durchführung des Verfahrens geeignete Vorrichtung.

**Ausführungsbeispiel**

Dem Simulationsexperiment liegt die folgende gedachte Ausgangssituation zugrunde.

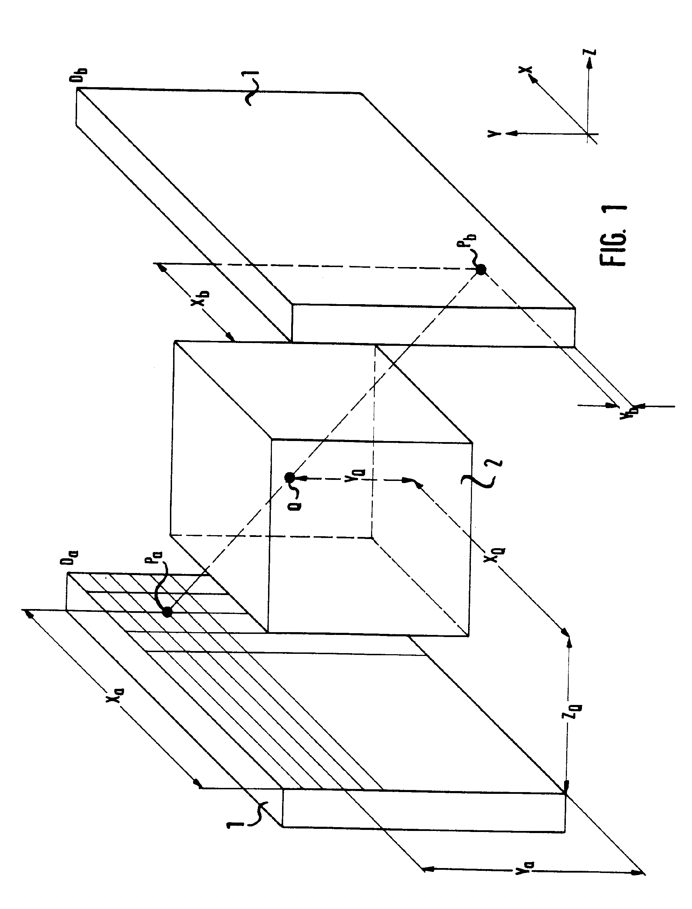

Ein menschliches Körperteil 2 befindet sich gem. Fig. 1 zwischen den zwei ortsauflösenden Photomultipliern 1. Zerfällt nun beispielsweise im Punkt Q ein Positron und lösen die beiden bei diesem Vorgang entstandenen $\gamma$-Quanten an den Detektororten $(X_a, Y_a)$ und $(X_b, Y_b)$ zeitgleich je ein Eingangssignal aus, so bestimmt der Computer per Zufallsgenerator eine Zahl r mit $r_a < r < r_b$ und gemäß

$$X_Q = (1 - r) \cdot X_a + r \cdot X_b$$
$$Y_Q = (1 - r) \cdot Y_a + r \cdot Y_b$$
$$Z_Q = (1 - r) \cdot Z_a + r \cdot Z_b$$

eine Ereigniskoordinate $(X_Q, Y_Q, Z_Q)$. Gegebenenfalls werden diese Koordinaten noch in ein raumfestes Koordinatensystem transformiert. $r_a$ und $r_b$ sind so gewählte Konstanten, daß die Ereigniskoordinate im oder nahe beim menschlichen Gewebe liegt.

Gedanklich sei das menschliche Körperteil in eine fest vorgegebene Anzahl an Einheitsvolumenelementen (indiziert durch Zahlentripel $\underline{i} = (i_1, i_2, i_3)$, zerlegt. Jenes Einheitsvolumenelement, in dem die Ereigniskoordinate liegt, adressiert einen Speicherplatz im Computer. Durch Inkrementieren dieses Speicherplatzes wird ein solches Positronenzerfallsereignis abgelegt. Durch Akkumulation der Ereignisse entsteht eine verschmierte Ereignisdichteverteilung $B_i$.

Die heutzutage in der Positronenemissionstomographie eingesetzten Computer bewältigen die erforderlichen Rechenschritte im On-line-Betrieb, solange die Zählrate die Größenordnung $10^5$ je Sekunde nicht wesentlich übersteigt.

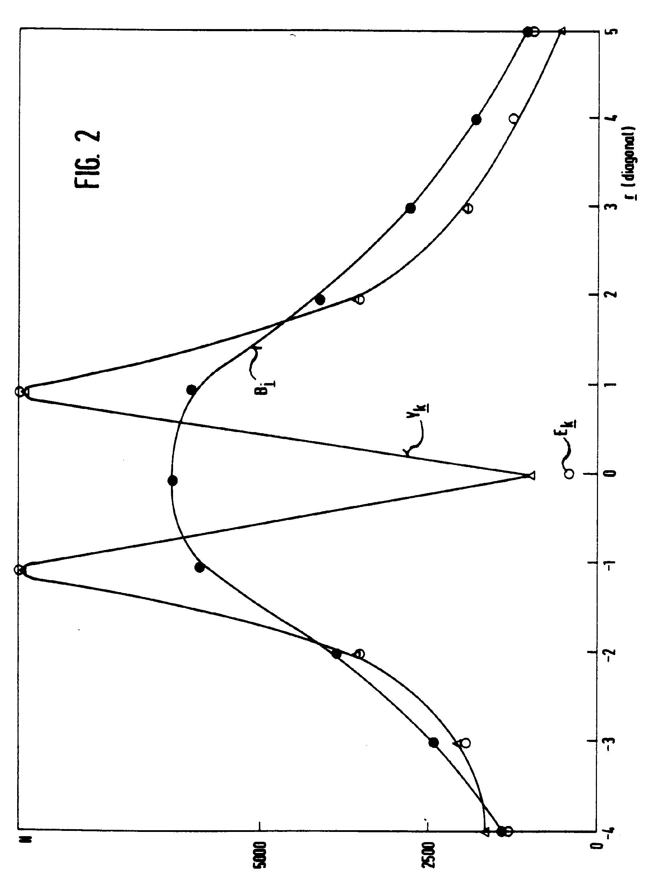

Ist das menschliche Körperteil gedanklich in 1000 Einheitsvolumenelemente zerlegt und werden Positronenzerfallsereignisse durch Drehen der Detektoren um die y-Achse bzw. vertikales Verschieben entlang der y-Achse von einem Raumwinkel zwischen 1 und 2 aus erfaßt, so entsteht ein in Fig. 2 dargestelltes, simuliertes Meßergebnis $B_i$. Der ebenfalls in Fig. 2 gezeigte, zugehörige wahre Lösungsvektor $E_k$ ist gemäß

$$\sum_k A_{\underline{i}\,\underline{k}} \cdot E_{\underline{k}} = B_{\underline{i}}$$

mit der verschmierten Ereignisdichteverteilung verknüpft. Die Auflösungsmatrix $A_{i\,k}$ ist dabei die akkumulierte Ereignisdichte, wenn alle Zerstrahlungen an einem einzigen Punkt Q auftreten. $A_{i\,k}$ wurde im Rahmen des Simulationsexperimentes analytisch berechnet.

Um aus den Meßwerten $B_i$ die gesuchte Funktion $E_k$ zu ermitteln, wurde wie folgt vorgegangen: Die Komponenten des n + 1 Vektors $Y^{(n+1)}$ wurden komponentenweise aus dem n-ten Vektor $Y^{(n)}$ gemäß

$$Y_{\underline{k}}^{(n+1)} = \alpha \, Y_{\underline{k}}^{(n)} \left( \frac{B_{\underline{k}}}{\sum_{\underline{j}} A_{\underline{k}\,\underline{j}} \, Y_{\underline{j}}^{(n)}} \right)^{\eta}$$

berechnet, wobei zunächst $\alpha = 1$ gesetzt war. Die Folge begann mit dem Vektor

$$Y_{\underline{k}}^{(1)} = \frac{|B_{\underline{k}}|}{|\sum_{\underline{j}} A_{\underline{k}\underline{j}} B_{\underline{j}}|} B_{\underline{k}} \quad .$$

Dabei ist | | die euklidische Vektornorm, also z.B.

$$|B_{\underline{k}}| = \left(\sum_{\underline{k}} B_{\underline{k}}^2\right)^{1/2}$$

Die Summe

$$S_{\underline{k}} = \sum_{\underline{j}} A_{\underline{k}\underline{j}} \cdot Y_{\underline{j}}^{(n)}$$

wurde näherungsweise in eine Summation über die diagonalen Elemente und in eine Mittelwertsbildung über die verbleibenden Elemente zerlegt d.h.

$$S_{\underline{k}} = \sum_{|\underline{j}-\underline{k}| \leq m} A_{\underline{k}\underline{j}} \cdot Y_{\underline{j}}^{(n)} + \sum_{|\underline{j}-\underline{k}| > m} \langle A_{\underline{k}} \rangle \cdot Y_{\underline{j}}^{(n)}$$

wobei

$$\langle A_{\underline{k}} \rangle = \frac{\sum_{|\underline{j}-\underline{k}| > m} A_{\underline{k}\underline{j}}}{\sum_{|\underline{j}-\underline{k}| > m} 1}$$

und m = 3 gesetzt worden ist.

Dieses Iterationsverfahren wurde abgegrochen, als für den n + 1 Lösungsvektor die Abweichung

$$\left| \sum_{\underline{k}} A_{\underline{i}\underline{k}} \cdot Y_{\underline{k}}^{(n+1)} - B_{\underline{i}} \right| \leq \varepsilon \cdot |B_{\underline{i}}|$$

war.

Mit dem Exponenten $\eta$ wurde die Zahl der Iterationsschritte beeinflußt.

Der Normierungsfaktor $\alpha$ wurde nachfolgend so gewählt, daß für die Normen der Vektoren

$$\left| \sum_{k} A_{\underline{i}\,\underline{k}} \cdot Y_{\underline{k}}^{(n+1)} \right| = \left| B_{\underline{i}} \right|$$

galt.

Zwischen 20 und 50 Iterationsschritte waren erforderlich, um die Schranke $\varepsilon = 1 \times 10^{-4}$ zu erreichen.

In Fig. 2 ist der so berechnete Lösungsvektor $Y_k$ dargestellt. Die relative Abweichung zum wahren Lösungsvektor lag in der Größenordnung $10^{-3}$.

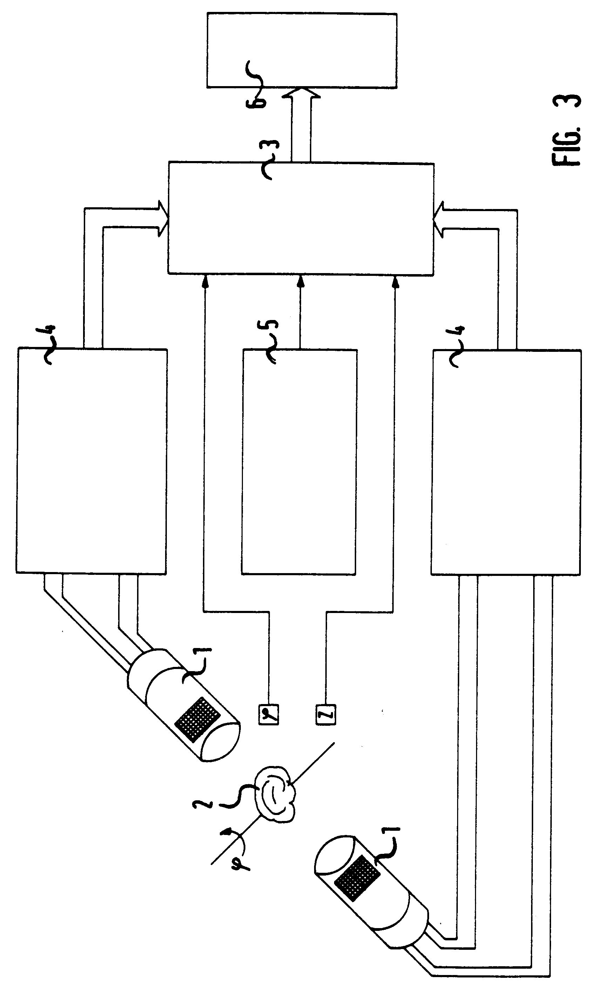

Figur 3 verdeutlicht die Vorrichtung zur Durchführung des Verfahrens. Die Photomultiplier 1 sind zueinander parallel ausgerichtet. Zwischen diesen befindet sich das Meßobjekt 2. Die Photomultiplier 1 können um das Meßobjekt 2 gedreht und vertikal verschoben werden. Über Datenleitungen wird dem Rechenwerk 3 jede Drehung $\phi$ bzw. Verschiebung Z mitgeteilt. Die Signalverarbeitung 4 dient der Übermittlung der Signale der Photomultiplier 1 an das Rechenwerk 3. Im Rechenwerk 3 werden zeitgleich eintreffende Signale in Flugbahnen umgerechnet, so daß dadurch Ortsinformationen über die Dichteverteilung der Positronen vorliegen. Der Zufallsgenerator 5 ist mit dem Rechenwerk 3 so verbunden, daß dieser durch Zufall einen Teil dieser Ortsinformationen auswählt. Der mit dem Rechenwerk 3 verbundene Speicher 6 dient der Speicherung der ausgewählten Ortsinformationen. Die gespeicherten Ortsinformationen werden nach Beendigung der Messung vom Rechenwerk verfahrensgemäß ausgewertet.

**Patentansprüche**

1. Verfahren zur Ermittlung der Dichteverteilung von ins menschliche Gewebe gebrachten und dort in je zwei entgegengesetzt fliegende $\gamma$-Quanten zerfallende Positronen, bei dem
   - zwei gegenübergestellte, den Menschen (2) einschließende $\gamma$-Detektoren derartige $\gamma$-Quanten signalisieren,
   - mittels eines an die $\gamma$-Detektoren angeschlossenen Computers ein gleichzeitiges derartiges Signalisieren eines jeden $\gamma$-Detektors und daher zwei $\gamma$-Quanten, die aus demselben Positronenzerfall stammen, registriert werden,
   - mittels des Computers im Fall der Registrierung solcher aus demselben Positronenzerfall stammenden zwei $\gamma$-Quanten die Flugbahn der zwei $\gamma$- Quanten berechnet und damit eine Ortsinformation über das zugehörige zerfallene Positron ermittelt und im Computer solche Ortsinformationen gespeichert werden,
   - die Positionen der $\gamma$-Detektoren derart verändert werden, daß aus der dann resultierenden Vielzahl solcher Ortsinformationen eine Verteilungsfunktion ensteht und
   - mittels des Computers aus dieser Verteilungsfunktion die Dichteverteilung der zerfallenen Positronen ermittelt wird,

   **dadurch gekennzeichnet,** daß
   - als $\gamma$-Detektoren zueinander parallel ausgerichtete ortsauflösende Photomultiplier (1) eingesetzt werden und
   - dem Speicher (6) des Computers nur ein per Zufallsgenerator (5) ausgewählter Teil an Ortsinformationen zugeführt wird, wobei dieser Teil an Ortsinformationen so groß gewählt ist, daß bei einer Online-Datenspeicherung dieser Ortsinformationen die Leistungsfähigkeit des Computers nicht überschritten wird.

2. Verfahren nach Anspruch 1,
   **dadurch gekennzeichnet,** daß
   - pro durch den Computer berechneter Flugbahn per Zufallsgenerator eine Koordinate dieser Flugbahn, hier Ereigniskoordinate genannt, willkürlich gewählt wird, wobei die zugelassenen Zufallsgeneratorzahlen so eingeschränkt werden, daß die Ereigniskoordinaten im oder nahe beim menschlichen Gewebe liegen.

3. Verfahren nach Anspruch 2,
   **dadurch gekennzeichnet,**
   daß der Ereigniskoordinatenraum in einzelne Raumelemente zerlegt wird, jedes Raumelement einer Speicherplatzadresse zugeordnet wird, eine Ereigniskoordinate in einem solchen Raumelement durch Inkrementierung um eins des zugeordneten Speicherplatzes gespeichert wird und so eine gespeicherte Ereigniskoordinatenverteilung $B_i$ entsteht, daß das Auflösungsvermögen $A_{ik}$ bei dieser Vorgehensweise experimentell, durch Computersimulation oder näherungsweise analytisch ermittelt wird und gemäß

$$\sum_k A_{ik} \cdot E_k = B_i$$

die Dichteverteilung $E_k$ der zerfallenen Positronen berechnet wird.

4. Verfahren nach Anspruch 3,
**dadurch gekennzeichnet**,
daß die Dichteverteilung der zerfallenen Positronen $E_k$ iterativ gemäß

$$Y_k^{(n+1)} = \alpha \cdot Y_k^{(n)} \cdot \left( \frac{B_k}{\sum_j A_{kj} \cdot Y_j^{(n)}} \right)^{\eta}$$

$0,1 < \eta < 10$
beginnend mit

$$Y_k^{(1)} = \frac{|B_k|}{\left| \sum_j A_{kj} \cdot B_j \right|} B_k$$

berechnet wird.

5. Verfahren nach Anspruch 4,
**dadurch gekennzeichnet**,
daß die Summe

$$S_k = \sum_j A_{kj} \cdot Y_j^{(n)}$$

näherungsweise in eine Summation über die diagonalen Elemente und in eine Mittelwertbildung über die verbleibenden Elemente zerlegt wird, d.h.

$$S_k = \sum_{|j-k| \leq m} A_{kj} Y_j^{(n)} + \sum_{|j-k| > m} \langle A_k \rangle Y_j^{(n)}$$

wobei

$$\langle A_{\underline{k}} \rangle = \frac{\sum_{|\underline{j}-\underline{k}|>m} A_{\underline{k}\underline{j}}}{\sum_{|\underline{j}-\underline{k}|>m} 1}$$

6. Vorrichtung zur Ermittlung der Dichteverteilung von ins menschliche Gewebe gebrachten und dort in je zwei entgegengesetzt fliegende $\gamma$-Quanten zerfallenden Positronen - bestehend aus zwei gegenübergestellten, den Menschen (2) so einschließenden $\gamma$-Detektoren, daß die $\gamma$-Quanten mittels Detektoren signa-lisierbar sind - einem derart mit den Detektoren verbundenen Rechenwerk (3), daß ein zeitgleiches Si-gnalisieren eines $\gamma$-Quants je Detektor mittels Rechenwert so registrierbar ist, daß die Signale durch das Rechenwerk in die zugehörige Flugbahn umrechenbar sind - einem mit dem Rechenwerk derart verbundenen Speicher (6), daß berechnete Flugbahnen als Ortsinformationen im Speicher (5) zwischenzeitlich speicherbar und für die Auswertung durch das Rechenwerk (3) abrufbar sind,
**dadurch gekennzeichnet,**
daß als Detektoren zueinander parallel ausgerichtete, ortsauflösende Photomultiplier (1) eingesetzt sind und daß ein Zufallsgenerator (5) derart mit dem Rechenwerk (3) verbunden ist, daß durch den Zufallsgenerator (3) ein Teil der Ortsinformationen auswählbar ist, so daß lediglich ein Teil der Ortsinformationen im Speicher (6) on-line speicherbar ist.

## Claims

1. Method for determining the density distribution of positrons which are introduced into human tissue and which decay therein into two oppositely travelling $\gamma$-quanta, in which method
   - two opposite $\gamma$-detectors enclosing the human (2) indicate such $\gamma$-quanta,
   - a simultaneous such indication of each respective $\gamma$-detector and accordingly two $\gamma$-quanta which originate from the same positron decay are recorded by means of a computer connected to the $\gamma$-detectors,
   - in the event of the recording of such two $\gamma$-quanta originating from the same positron decay, the path of the two $\gamma$-quanta is computed by means of the computer and thus a location message concerning the pertinent decayed positron is determined and such location messages are stored in the computer,
   - the positions of the $\gamma$-detectors are altered in such a manner that a distribution function is formed from the then resulting multiplicity of such location messages, and
   - the density distribution of the decayed positrons is determined from the distribution function by means of the computer,
   characterized in that
   - spatially resolving photomultipliers (1) aligned parallel to one another are used as $\gamma$-detectors, and
   - only a part, selected by random sequence generator (5), of location messages is fed to the memory (6) of the computer, this part of location messages being selected to be of such magnitude that in the case of an on-line data storage of these location messages, the capacity of the computer is not exceeded.

2. Method according to Claim 1,
   characterized in that
      - for each path computed by the computer, a coordinate of this path, here referred to as the event coordinate, is arbitrarily selected by random sequence generator, the permitted random sequence generator numbers being restricted so that the event coordinates lie within or close to the human tissue.

3. Method according to Claim 2,
   characterized in that the event coordinate space is split into individual spatial elements, each spatial element is allocated to a memory location address, and an event coordinate in such a spatial element is stored by incrementation by one of the allocated memory location and thus a stored event coordinate distribution $B_l$ is formed, in that the resolution $A_{lk}$ in this procedure is determined experimentally, by computer simulation or, by way of approximation, analytically, and the density distribution $E_k$ of the decayed positrons is computed in accordance with

$$\sum_k A_{lk} \cdot E_k = B_l$$

.

4. Method according to Claim 3,
characterized in that the density distribution of the decayed positrons $E_k$ is computed iteratively in accordance with

$$Y_k^{(n+1)} = \alpha \cdot Y_k^{(n)} \cdot \left( \frac{B_k}{\sum_j A_{kj} \cdot Y_j^{(n)}} \right)^{\eta}$$

$0.1 < \eta < 10$
commencing with

$$Y_k^{(1)} = \frac{|B_k|}{\left| \sum_j A_{kj} \cdot B_j \right|} \, B_k$$

5. Method according to Claim 4,
characterized in that the sum

$$S_k = \sum_j A_{kj} \cdot Y_j^{(n)}$$

is split, by way of approximation, into a summation over the diagonal elements and into a mean-value formation over the remaining elements, i.e.

$$S_k = \sum_{|j-k| \le m} A_{kj} Y_j^{(n)} + \sum_{|j-k| > m} \langle A_k \rangle Y_j^{(n)}$$

where

$$\langle A_k \rangle = \frac{\sum_{|j-k| > m} A_{kj}}{\sum_{|j-k| > m} 1}$$

.

**6.** Apparatus for determining the density distribution of positrons which are introduced into human tissue and which decay therein into in each case two oppositely travelling γ-quanta - comprising two opposite γ-detectors enclosing the human (2) such that the γ-quanta can be indicated by means of detectors - an arithmetic-logic unit (3) connected to the detectors in such a manner that a contemporaneous indication of a γ-quantum for each detector can be recorded by means of the arithmetic-logic unit, so that the signals can be converted by the arithmetic-logic unit into the pertinent path - a memory (6) connected to the arithmetic-logic unit, in such a manner that computed paths can in the meantime be stored in the memory (6) as location messages and can be called up for evaluation by the arithmetic-logic unit (3), characterized in that spatially resolving photomultipliers (1) aligned parallel to one another are used as detectors, and in that a random sequence generator (5) is connected to the arithmetic-logic unit (3) in such a manner that a part of the location messages can be selected by the random sequence generator (5), so that only a part of the location messages can be stored on-line in the memory (6).

## Revendications

**1.** Procédé pour déterminer la distribution de densité de positrons qui sont insérés dans le tissu humain et s'y désintègrent en respectivement deux quanta γ circulant en des sens opposés, selon lequel
- deux détecteurs γ, qui sont disposés en vis-à-vis et enserrent entre eux l'homme (2), signalent de tels quanta γ,
- au moyen d'un ordinateur raccordé aux détecteurs γ, une telle signalisation simultanée de chaque détecteur γ et par conséquent deux quanta γ, qui proviennent de la même désintégration d'un positron, sont enregistrés,
- à l'aide de l'ordinateur, dans le cas de l'enregistrement de deux quanta γ de ce type, qui proviennent de la même désintégration d'un positron, la trajectoire de vol des deux quanta γ est calculée et par conséquent une information de lieu concernant le positron désintégré associé est déterminée et de telles informations de lieu sont mémorisées dans l'ordinateur,
- les positions des détecteurs γ sont modifiés de telle sorte qu'on obtient une fonction de distribution à partir de la multiplicité, que l'on obtient alors, de telles informations de lieu, et
- à l'aide de l'ordinateur, la distribution de densité des positrons désintégrés est déterminée à partir de cette fonction de distribution,
caractérisé en ce que
- on utilise, comme détecteurs γ, des photomultiplicateurs (1) à résolution locale, qui sont orientés parallèlement l'un à l'autre,
- seule une partie des informations de lieu, qui est sélectionnée pour chaque générateur de signaux aléatoires (5) est envoyée à la mémoire (6) de l'ordinateur, cette partie d'informations de lieu étant choisie suffisamment grande pour que, dans le cas d'une mémorisation de données en direct de ces informations de lieu, les capacités potentielles de l'ordinateur ne soient pas dépassées.

**2.** Procédé selon la revendication 1, caractérisé en ce que
- pour chaque trajectoire de vol, calculée par l'ordinateur, pour chaque générateur de signaux aléatoires, une coordonnée de cette trajectoire de vol, ici désignée sous l'expression coordonnée de résultat, est sélectionnée à volonté, les nombres autorisés de générateurs de signaux aléatoires étant limités de manière que les coordonnées de résultat soient situées dans ou à proximité du tissu humain.

**3.** Procédé suivant la revendication 2, caractérisé en ce que l'espace des coordonnées de résultat est subdivisé en éléments individuels d'espace, chaque élément d'espace est associé à une adresse dans l'emplacement de mémoire, une coordonnée de résultat est mémorisée dans un tel élément d'espace par incrémentation de un de l'emplacement de mémoire associé et on obtient ainsi une distribution mémorisée $B_i$ de coordonnées de résultat, et que le pouvoir de résolution $A_{ik}$ est déterminé expérimentalement lors de ce mode opératoire, par simulation sur ordinateur ou analytiquement de façon approximative, et que la distribution de densité $E_k$ des positrons désintégrés est calculée conformément à

$$\sum_{k} A_{ik} . E_k = B_i .$$

**4.** Procédé selon la revendication 3, caractérisé en ce que ladite impulsion de densité des positrons désin-

tégrés $E_{\underline{k}}$ est calculée de façon itérative conformément à

$$Y_{\underline{k}}^{(n+1)} = \alpha \cdot Y_{\underline{k}}^{(n)} \cdot \left( \frac{B_{\underline{k}}}{\sum_{\underline{j}} H_{\underline{k}\underline{j}} \cdot Y_{\underline{j}}^{(n)}} \right)^{\eta}$$

$0,1 < \eta < 10$ commençant par

$$Y_{\underline{k}}^{(1)} = \frac{\left| B_{\underline{k}} \right|}{\left| \sum_{\underline{j}} H_{\underline{k}\underline{j}} \cdot B_{\underline{j}} \right|} \quad B_{\underline{k}}$$

5. Procédé selon la revendication 4, caractérisé en ce que la somme

$$S_{\underline{k}} = \sum_{\underline{j}} A_{\underline{k}\underline{j}} \cdot Y_{\underline{j}}^{(n)}$$

est subdivisée approximativement en une sommation sur les éléments diagonoaux et en une formation de la valeur moyenne sur les autres éléments, c'est-à-dire

$$S_{\underline{k}} = \sum_{|\underline{j}-\underline{k}| \leq m} H_{\underline{k}\underline{j}} Y_{\underline{j}}^{(n)} + \sum_{|\underline{j}-\underline{k}| > m} \langle \tilde{H}_{\underline{k}} \rangle Y_{\underline{j}}^{(n)}$$

avec

$$\langle H_{\underline{k}} \rangle = \frac{\sum_{|\underline{j}-\underline{k}| > m} H_{\underline{k}\underline{j}}}{\sum_{|\underline{j}-\underline{k}| > m} 1} \quad .$$

6. Dispositif pour déterminer la distribution de densité de positrons introduits dans le tissu humain et désintégrés dans ce dernier sous la forme respectivement de deux quanta γ circulant en des sens opposés - constitué par deux détecteurs γ, qui sont disposés en vis-à-vis et enserrent l'homme (2) de telle sorte que les quanta γ peuvent être signalés au moyen de détecteurs - par une unité de calcul (3) reliée au détecteur de telle sorte qu'une signalisation simultanée d'un quanta γ pour chaque détecteur peut être enregistrée par l'unité de calcul (3)

de telle sorte que les signaux peuvent être convertis par l'unité de calcul en la trajectoire de vol associée - par une mémoire (6) reliée à l'unité de calcul de telle sorte que les trajectoires de vol calculés peuvent être mémorisés temporairement en tant qu'informations de lieu dans la mémoire (6) et peuvent être appelées, en vue de l'évaluation, par l'unité de calcul (3),
caractérisé en ce qu'on utilise, comme détecteurs, deux photomultiplicateurs (1) à résolution locale, orientés parallèlement l'un à l'autre et qu'un générateur de signaux aléatoires (5) est relié à l'unité de calcul (3) de telle sorte qu'une partie des informations de lieu peut être sélectionnée par le générateur de signaux aléatoires (5) de sorte que seule une partie des informations de lieu peuvent être mémorisées en direct dans une mémoire (6).

FIG. 1

FIG. 2

EP 0 537 762 B1

FIG. 3